# EUROPEAN PATENT APPLICATION

(11) **EP 4 066 942 A1**
(43) Date of publication of application: **05.10.2022**
(21) Application number: 20894828.1
(22) Date of filing: 05.11.2020
(51) Int. Cl.: B01L 3/00, C12M 1/00, C12N 5/09

(54) **METHOD FOR USING MICROFLUIDIC CHIP, AND DEVICE THEREOF**

(30) Priority: 25.11.2019 CN 201911168937
(71) Applicant: HANGZHOU WEIZHU BIOLOGICAL TECHNOLOGY CO., LTD., Zhejiang 310052 (CN)
(72) Inventor: YANG, Chaoyong, Hangzhou, Zhejiang 310052 (CN); ZHANG, Mingxia, Hangzhou, Zhejiang 310052 (CN); ZOU, Yuan, Hangzhou, Zhejiang 310052 (CN)
(74) Representative: Osterhoff, Utz
(86) International application number: PCT/CN2020/126616
(87) International publication number: WO 2021/103970

(57) **Abstract**

The present invention relates to a method of using a microfluidic chip comprising introducing a gas into the microfluidic chip to replace the liquid that has been introduced into the microfluidic chip and forming a micro-reaction chamber in the form of a liquid-in-gas in the microfluidic chip. The present invention also relates to a method for obtaining assay data, a computer program product embodied in a computer-readable medium and a kit. The methods described in the present invention are easy to operate, low cost, versatile, enabling rapid exchange of fluids, achieving efficient separation and capture of single particles with high purity. In addition, the methods can avoid clogging the chip and facilitate recycling.

## Description

### TECHNICAL

This invention relates to the field of microfluidics and molecular biology, particularly to methods of using microfluidic chips.

### BACKGROUND

Cells are the basic units of biological and life activities. The analyzing and processing of the average signals of a large number of cells by traditional methods allows the averaging of the signals to obscure the understanding of the heterogeneity among the various living systems of the human body and the cells that make up these systems. With the development of high-throughput sequencing technology, single-cell/particle sequencing technology has become one of the most important tools for single-cell analysis, which has greatly improved the efficiency and accuracy of single-cell/particle analysis.

Microfluidic chips integrate the basic operating units of sample preparation, reaction, isolation, detection, and cell culture, sorting, and lysis in biology, chemistry, and other related fields onto microchips, forming networks by microchannels with controlled fluids running throughout the system, enabling miniaturization and integration of conventional chemical or biological experiments. Microfluidic chips are particularly suitable for single-cell/particle analysis studies due to their low consumption of reaction reagents and the short action time.

Analysis platforms of microfluidic chips in the art often require the addition of various reagents within the chip channels to achieve proper performances of multiple biochemical reactions. After the addition of different reagents, an oil phase (i.e., oil sealing) is often used to isolate the continuous reaction chamber to prevent signal interference between adjacent reaction units to achieve high-throughput single-cell analysis. However, due to the natural property of the incompatibility of oil and water, the use of the oil phase often leads to the following problems which make it difficult to achieve the integration of high-throughput and multi-reaction processing on a chip simultaneously: the oil-water droplets forms unstably, making the subsequent cell fusion and lysis to be difficult; the oil phase remains in the chip channel and clogs, making the chip difficult to reuse; the formed oil-water droplets have poor encapsulation and demulsification effect; high cost, low recovery efficiency, etc.

### SUMMARY

To solve the above-mentioned problems in the prior art, the present invention aims to provide methods for using microfluidic chips. The methods described in the present invention are easy to operate, low cost, and versatile, can realize rapid exchanges of fluids, and achieve efficient separation and capture of single particles with high purity. The methods can also avoid clogging the chips and facilitate recycling.

The present invention achieves the above objectives by the following technical solutions.

A method of using a microfluidic chip comprising introducing a gas into the microfluidic chip to replace the liquid that has been injected into the microfluidic chip, forming one or more micro-reaction chambers in the form of a liquid-in-gas in the microfluidic chip.

In some embodiments of the present invention, the liquid that has been injected into the microfluidic chip contains particles. In some embodiments of the present invention, the liquid that has been injected into the microfluidic chip does not contain particles.

In some embodiments of the present invention, the micro-reaction chambers contain particles. In other embodiments of the present invention, the micro-reaction chambers do not contain particles.

The particles described in the present invention, are particles known in the art, which need to be captured, analyzed, and reacted. The particles have a particle size in the range of 5 µm to 1000 µm, such as 5 µm - 200 µm. Particles known in the art include, but are not limited to, cells, cell clusters, microorganisms, microbial clusters, phages, exosomes, micelles, and artificial microspheres, wherein the artificial microspheres include, but are not limited to, microspheres such as polyethylene glycol, polyacrylamide, polymethacrylic acid, polymethacrylate, polyvinyl alcohol, polyethylene, polystyrene, polyesters (e.g., PLGA and PLA), silica, and graphene, where the artificial microspheres contain on their surface substances that achieve the intended detection purpose, such as but not limited to, compounds such as nucleic acid aptamers, and biological macromolecules such as nucleic acids, proteins, and peptides. In some embodiments, the artificial microspheres are microspheres modified with nucleic acid sequences for RNA capture. In other embodiments, the artificial microspheres are microspheres modified with nucleic acid sequences for gene capture. In other embodiments, the artificial microspheres are microspheres modified with molecules such as nucleic acid aptamers or antibodies. In other embodiments, the artificial microspheres are microspheres modified with two or more of these molecules of microspheres.

In some embodiments, the micro-reaction chamber contains a single microsphere. In some embodiments, the micro-reaction chamber contains a single cell. In some embodiments, the micro-chamber is free of particles.

In some embodiments, the gas may be any gas conventionally used in the art, including but not limited to air, nitrogen, oxygen, helium, hydrogen, carbon dioxide, neon, argon, xenon, and the like. These gases can be used alone or in combination. Those skilled in the art can choose the gas to be used according to the requirements for the compatibility of actual biochemical reactions.

In some embodiments, the gas is introduced into the microfluidic chip through the same inlet as the liquid that has been injected into the microfluidic chip. In some embodiments, the gas is introduced into the microfluidic chip through different inlets than the liquid that has been injected into the microfluidic chip. In some embodiments, the gas is introduced into the microfluidic chip through different inlets.

In embodiments of the present invention, when a gas is introduced from one opening of the microfluidic chip, the liquid that has been injected into the microfluidic chip is replaced by the gas and flows out from another opening.

The gas of the present invention may be introduced into the microfluidic chip via a gas cylinder through a gas line. In some embodiments, the same gas cylinder may be used to introduce gas to the same gas-phase inlet or a different gas-phase inlet. In other embodiments, different gas cylinders may be used to introduce gas to the same gas-phase inlet or a different gas-phase inlet. Those skilled in the art know the conventional methods of using gas cylinders and connecting piping.

In some embodiments, the gas is introduced into the microfluidic chip at a gas flow rate of 0.02 L/min to 1.0 L/min. In some embodiments, the gas is introduced into the microfluidic chip at a gas flow rate of 0.05 L/min to 0.7 L/min. In some embodiments, the gas is introduced into the microfluidic chip at a gas flow rate of 0.06 L/min, 0.07 L/min, 0.08 L/min, 0.09 L/min, or 0.1 L/min.

In the present invention, the time required for the entering of the gas can be adjusted according to practical needs so that the gas replaces, in whole or in part, the liquid that has been injected into the microfluidic chip. In some embodiments, the time required for the entering of the gas is 10 min to 90 min. In some embodiments, the time required for the entering of the gas is 10 min to 40 min. In some embodiments, the time required for the entering of the gas is 15 min to 25 min.

The microfluidic chips described in the present invention may be conventionally used in the field. For example, a variety of microfluidic chips exemplified in the review literature (Shembekar, Chaipan, et al. 2016) or textbook (The Micro-Nano-Fluidic Chip Laboratory, by Bing Seung Lin) may be applicable to the method of using microfluidic chips provided by the present invention (i.e., the gas sealing method). Further, the method of the present invention is particularly applicable to chips similar to the microfluidic chip described in the patent application CN107012067A, wherein the inlet of the oil phase can be replaced with a gas phase inlet.

In some embodiments, the microfluidic chip comprises three parts: a capture layer, a control layer, and a slide. The capture layer includes two parallel sets of particle capture flow channels and a connecting channel that connect them. The particle capture flow channels contain a plurality of single particle capture units, wherein each unit includes a flow channel, a reservoir chamber, and a capture channel. The diameter of the capture channel is smaller than that of the particles to be captured, allowing a single particle to be captured. The left end of the flow channel of the former capture unit is connected to the right end of the flow channel of the latter capture unit. The reservoir chamber is located between the two ends of the flow channel and has three channels. The first channel leads to the fluid inlet end of the flow channel, having a diameter greater than that of the single particle to be captured. The second channel is the capture channel, which leads to the liquid outlet end of the flow channel, having a diameter smaller than that of the single particle to be captured. The third channel is the connecting channel, which leads to the reservoir chamber of the other capture unit in parallel, having a diameter smaller than that of the single particle to be captured. The connecting channel connects the reservoir chambers of the two paired particle capture units.

Each capture flow channel is also provided with a particle inlet at the front, a gas phase inlet at the end, and a particle outlet at the end, respectively.

The dimensions (length, width, height) of the two sets of particle capture flow channels and the shape and size of the capture units therein may be the same or different.

The control layer includes a blocking channel directly below or above the connecting channels of the capture layer, separated by a membrane therebetween. The blocking channel is provided with an inlet.

The capture layer and the control layer are located above the slide.

In some embodiments, the microfluidic chip further includes a driving pump unit for changing the volume of the reservoir chamber of the particle capture unit, including a driving pump control network channel and a pump deformation chamber in communication, and the driving pump control network channel is also provided with a driving pump inlet. The driving pump deformation chamber is located above or below the reservoir chamber of the single particle capture unit of the capture layer and is separated from the reservoir chamber by the membrane.

In some embodiments, the size of the channel is determined by the size of the particles to be specifically used and analyzed. Generally, the width of the capture flow channel may be 5-500 µm, for example, including but not limited to 5-100 µm, 100-300 µm, 300-500 µm, 5-50 µm, 50-200 µm, 200-400 µm, 10 µm, 20 µm, 50 µm, 80 µm, 100 µm, 200 µm, 300 µm, or 400 µm. The depth of the capture flow channel may be 5-500 µm, for example, including, but not limited to, 5-100 µm, 100-300 µm, 300-500 µm, 5-50 µm, 50-200 µm, 200-400 µm, 10 µm, 20 µm, 50 µm, 80 µm, 100 µm, 200 µm, 300 µm, or 400 µm. The width of the connecting channel may be 3-100 µm, for example, including but not limited to 3-30 µm, 30-50 µm, 50-100 µm, 10-40 µm, 40-80 µm, 5 µm, 10 µm, 20 µm, 50 µm, 60 µm, 70 µm, 80 µm, or 90 µm. The depth of the connection channel may be 3-100 µm, for example: including but not limited to 3-30 µm, 30-50 µm, 50-100 µm, 10-40 µm, 40-80 µm, 5 µm, 10 µm, 20 µm, 50 µm, 60 µm, 70 µm, 80 µm, or 90 µm.

In some embodiments, the particles have a diameter of 5 to 200 µm and the particles may be any microsphere or cell of micron size. It is possible to use the microfluidic chip for pairing between microspheres, between cells, or between microspheres and cells.

In some embodiments, the microspheres may have a diameter of 5-200 µm, for example, including but not limited to 5 µm, 10 µm, 15 µm, 20 µm, 30 µm, 70 µm, 80 µm, 90 µm, 100 µm, 120 µm, 150 µm, or 180 µm.

In some embodiments, the cells may have a diameter of 5-100 µm, for example, including, but not limited to, 5 µm, 10 µm, 15 µm, 20 µm, 30 µm, 70 µm, 80 µm, or 90 µm.

In some embodiments, the material of the capture layer and the slide used in the microfluidic chip of the present invention can be a silicon wafer, glass, polymethyl methacrylate, polyethylene, polypropylene, polyvinyl chloride, polyester, and the like. The material of the control layer may be polymethyl methacrylate, polyethylene, polypropylene, polyvinyl chloride, polyester, and the like.

In some embodiments, the material of the capture and control layers can be polydimethylsiloxane (PDMS), and the material of the slide can be glass.

In some embodiments, the capture and control layers are reversibly bonded together by thermal calibration and then plasma bonded to the slide.

In some embodiments, the capture and the control layers are bonded together by plasma surface activation and then bonded to the slide by plasma.

In some embodiments, the particles are introduced at a flow rate of 0.005 mL/h-10 mL/h, for example, including but not limited to 0.005 mL/h-0.05 mL/h, 0.05 mL/h-0.1 mL/h, 0.1 mL/h-0.5 mL/h, 0.5 mL/h-2 mL/h, 2 mL/h-6 mL/h, 6 mL/h-10 mL/h, 0.05 mL/h-0.5 mL/h, 0.5 mL/h-3 mL/h, 3 mL/h-8 mL/h, 0.01 mL/h, 0.05 mL/h, 0.1 mL/h, 0.2 mL/h, 0.5 mL/h, 0.8 mL/h, 1 mL/h, 2 mL/h, 4 mL/h, 6 mL/h, or 8 mL/h.

In some embodiments, the blocking pump functions as a barrier to the connecting channel of the capture layer by deforming through the introduction of fluid. The fluid filled in the blocking pump can be an aqueous solution, oil, or air, which can be introduced by a syringe pump. By controlling the pressure of the syringe pump, the pressure of the blocking pump is controlled, that is, the degree of blocking to the connection channel of the capture layer is controlled.

In some embodiments, the basic workflow of the microfluidic chip is as follows:
Step A: Increasing the pressure of the blocking pump to deform the barrier channel, causing the membrane to squeeze the uppermost layer of the connecting channel until the connecting channel between two parallel sets of the particle capture flow channels is completely blocked; introducing a liquid containing particles from the inlet of one set of the particle capture flow channel. When a single particle enters the capture unit, the particle enters the reservoir chamber first because the path of the fluid through the flow channel is longer than that through the reservoir chamber. Since the width (tube diameter) of the capture channel is smaller than the diameter of the particle, the particle is stuck in front of the capture channel while blocking the capture channel, causing the flow rate of the fluid through the reservoir chamber to approach zero. At this point, subsequent particles cannot re-enter the reservoir chamber but only enter the next capture unit through the flow channel, thus achieving a single particle capture. This process is repeated in subsequent particle capture units, allowing for high throughput single-particle capture.

Step B: For the above-mentioned particle capture flow channel to achieve single-particle capture, introducing a gas via the gas phase inlet. When the gas enters the capture unit, it enters the capture channel without entering the reservoir chamber because the width of the capture channel is much smaller than that of the flow channel and resulting in higher capillary resistance. At that time, the liquid in the reservoir chamber is retained, forming a micro-reaction chamber in which the gas is encapsulated by the liquid. When the gas flows throughout the chip, all the capture units in the flow channel are isolated into individual droplets of liquid-in-gas, enabling the separation of individual liquid-in-gas micro-reaction chambers.

When the gas enters the capture unit, because the width of the capture channel is much smaller than that of the flow channel, its capillary resistance is larger, so the gas phase enters the capture channel without entering the reservoir chamber; at which time the liquid in the reservoir chamber is retained, forming a micro-reaction chamber in the form of a liquid-in-gas. As the gas flows throughout the chip, all the capture units in the particle capture flow channel are isolated into individual liquid-in-gas droplets, enabling the separation of individual liquid-in-gas micro-reaction chambers.

Optionally, in some embodiments, before said step B, the workflow may further comprise steps A-1 :
Step A-1: Introducing a liquid required to perform the next step through the sample inlet of the particle capture flow channel that achieves single-particle capture, for eluting the liquid containing residual particles in the flow channel.

In some embodiments, steps A-1 and/or B may be repeated as many times as practically necessary. The liquid and/or gas used may be the same or different each time steps A-1 and/or step B is performed.

In some embodiments, after step B, steps C, C-1, and D are performed similarly to steps A, A-1, and B, respectively, to enable high throughput single-particle capture for another set of the particle capture flow channel that has not yet undergone single-particle capture. In some embodiments, steps C-1 and/or D may be repeated as many times as practically necessary. The liquid and/or gas used may be the same or different each time steps C-1 and/or D are performed. Thereafter, the workflow further comprises:
Step E: reducing or shutting down the blocking pump. At this point, the connection channel between the two parallel sets of particle capture flow channels is opened. The liquid-in-gas micro-reaction chambers for pairing between the two sets of particle capture flow channels are in a state of interconnection. Due to the diffusion, the liquid-in-gas micro-reaction chambers in the two reservoir chambers can achieve mutual substance exchange. After a period of sufficient reaction, high-throughput independent reactions between the paired particles are achieved.

In some embodiments, in step E, a driving pump located at the bottom of the reservoir chamber of each of the capture units of the two sets of particle capture channels may be used to alternately change the volume of the reservoir chambers to facilitate the rapid exchange of substances in the reservoir chambers of the two capture units.

In some embodiments, after step E, any one or several steps of steps A-D above can be repeated, such as repeating step B and step A-1, or repeating steps A, B, and optionally A-1 and C, or repeating step B and optionally A-1 and step C, etc. By repeatedly replacing liquids and forming liquid-in-gas micro-reaction chambers, multi-functional operations such as the addition and replacement of a variety of reaction particles and reagents, and the formation of reaction chambers can be realized.

In some embodiments, the final step of the workflow is step F:
Step F: peeling off the capture layer from the control layer, and eluting the captured or reacted particles in the reservoir chambers exposed to the surface of the chip with a buffer solution for subsequent analytical studies.

The present invention also provides a microfluidic chip containing one or more micro-reaction chambers in the form of a liquid-in-gas. Wherein the one or more micro-reaction chambers in the form of a liquid-in-gas and the microfluidic chip are as defined above.

The present invention further provides a method for obtaining an assay data, comprising:
Loading a sample to be assayed into the microfluid chip and introducing a gas into the microfluid chip to form one or more micro-reaction chambers in the form of a liquid-in-gas, and obtaining the assay data by measurement.

The method of obtaining the assay data also includes comparing the assayed data with the corresponding data stored in a database (e.g., a standard curve), and obtaining the data for quantifying the assayed sample.

A computer program product embodied in a computer readable medium, when executed on a computer, the execution steps comprising:
controlling and introducing a gas into the microfluidic chip.

Another computer program product embodied in a computer-readable medium, when executed on a computer, the execution steps comprising: introducing a gas into the microfluidic chip loaded with a sample to be assayed, waiting for the formation of one or more micro-reaction chambers in the form of a liquid-in-gas, and then measuring and obtaining assay data for the sample.

The present invention also provides a kit comprising:
a microfluidic chip; and
a computer program product embodied in a computer readable medium.

The beneficial effects of the present invention are as follows:
1. The method of the present invention can stably realize the forming of a liquid-in-gas micro-reaction chamber, solving the problem that when the "oil sealing" method is used in microfluidic chips, the emulsion is difficult to break over the oil-water interface and the oil-water droplets cannot be formed effectively.
2. The gas used in the method of the present invention is cheap and easy to obtain, without batch differences, and the system is stable, thus solving the problems of expensive oil phases containing surfactants and unstable results due to large batch differences in the "oil sealing" method.
3. The method of the invention can easily remove the solution reagents from the microfluidic chip channel, avoiding the residue of microemulsion droplets in the microfluidic chip channel in the conventional "oil sealing" method, and allowing the replacement of new reagents for individual droplets, making it possible to add multiple reagents in sequence, greatly expanding the range of single-particle-dependent tests that can be performed on microfluidic chips, and improving the efficiency of experiments, testing, and analysis.
4. The conventional "oil sealing" method requires the use of emulsion breakers to break the emulsion of the captured particles in the form of a "water-in-oil", which is often ineffective and costly. The methods of the invention can effectively achieve "background deduction" and 100% non-destructive recovery of the captured particles, solving the problem of contamination of the particles caused by encapsulating with the oil in the conventional "oil sealing" method, and the high loss rate of microspheres caused by the extraction and recovery of microspheres.
5. In a microfluidic chip that allows the pairing of captured particles, the methods of the invention enable stable droplet mixing and reagent replacement and solve the problems of the low ratio of droplets that can be effectively paired and the inability of particles (e.g. cells) to be lysed caused by the difficulty in fusing the paired droplets due to the isolation of the paired droplets by oil film in the conventional "oil sealing" method.

### DESCRIPTION OF DRAWINGS

Figure 1 illustrates a top view of the overall structure of the microfluidic chip, where 1 and 2 are the sample inlets, 3 is the blocking pump inlet, 4 and 5 are the gas inlet, and 6 and 7 are the sample outlet.
Figure 2 illustrates a schematic diagram of the enlarged structure of the microfluidic chip, in which 8 and 9 are particle capture units, 10 and 11 are particle capture flow channels, 12 is the blocking channel, and 13 is the connection channel.
Figure 3 illustrates a top view of the particle capture unit, where 10 and 11 are the particle capture flow channels, 12 is the blocking channel, 13 is the connection channel, 14 and 15 are the reservoir chambers, and 16 and 17 are the particle capture channels, respectively.
Figure 4 illustrates a cross-sectional view of the particle capture unit, where 13 is the connecting channel, 14 and 15 are the reservoir chambers, 12 is the blocking channel, and 18 is the membrane.
Figure 5 illustrates a top view of the overall structure of the pressure-driving microfluidic chip, where 19 and 20 are the driving pump inlets.
Figure 6 illustrates a schematic diagram of the enlarged structure of the pressure-driving microfluidic chip, in which 21 and 22 are the driving pump control network channels.
Figure 7 illustrates a top view of the particle/cell pairing capture unit of the pressure-driving microfluidic chip, where 23 and 24 are the driving pump deformation chambers of the particle capture units.
Figure 8 illustrates a cross-sectional view of the particle/cell paired capture units of the pressure-driving microfluidic chip, where 25 and 26 are membranes.
Figure 9 illustrates micro-reaction chambers in the form of a water-in-gas as shown by the microscope after the gas is introduced into the microfluidic chip using the method of the present invention.
Figure 10 illustrates micro-reaction chambers in the form of water-in-gas (with particles) after the gas is introduced into the microfluidic chip using the method of the present invention.
Figure 11 illustrates a diagram of the mixing process of the paired micro-reaction chambers shown in the microscope fluorescence field after the gas is introduced into the microfluidic chip using the method of the present invention.
Figure 12 illustrates a schematic and microscopic view of the replacement of the solution inside the chip after the gas is introduced into the microfluidic chip using the method of the present invention.
Figure 13 illustrates a graph of the statistical results of the recovery of microspheres after capturing/reacting the microspheres using the method of the present invention (gas phase replacement) by introducing the gas into the chip compared to the prior art method (oil phase replacement).
Figure 14 illustrates a top microscopic view of the chip before and after the particles are captured, reacted, and recovered after the gas is introduced into the chip using the method of the present invention.
Figure 15 illustrates a microscopic view of the chip wherein water-in-oil micro-reaction chambers are formed when using the prior art oil phase.
Figure 16 illustrates a microscopic fluorescence imaging view of the chip wherein water-in-oil micro-reaction chambers are formed when using the prior art oil phase.
Figure 17 illustrates an on-chip microscopic view of the chip wherein water-in-oil micro-reaction chambers are formed when using the prior art oil phase.

### MODE OF CARRYING OUT THE INVENTION

### Example 1

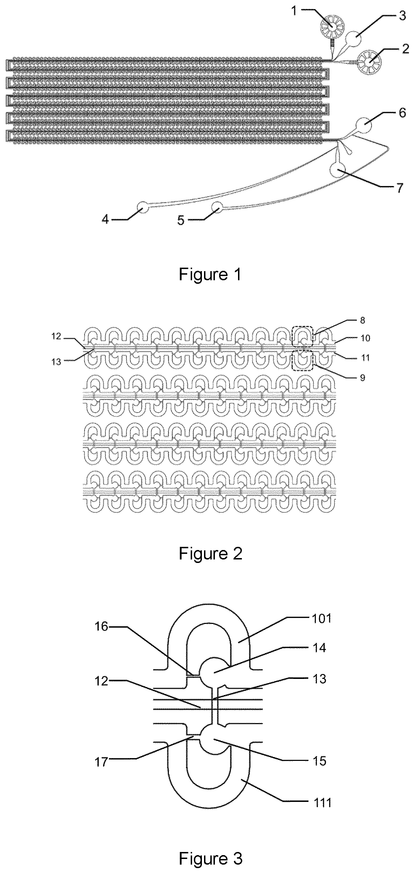

High throughput paired capture of single particles based on the methods of the present invention of using the microfluidic chips. The chip in used is shown in Figures 1-4. The chip which includes a capture layer, a control layer, and a slide is prepared by a standard soft lithography technique. The capture layer consists of a capture flow channel 10, a capture flow channel 11, and a connecting channel 13. The control layer consists of a blocking channel 12. The capture flow channel 10 further conclude a plurality of particle capture unit 8 connected in series at the beginning and end. Each unit consists of a flow channel 101, a reservoir chamber 14, and a capture channel 16. Referring to Figure 3, the flow channel 101 includes a U-shaped tube (in other embodiments, it may also be an arc or other zigzag shape), with the left arm end of the U-shaped tube of the former unit connecting to the right arm end of the U-shaped tube of the latter unit, and the connecting part between the U-shaped tubes being straight. The reservoir chamber 14 is located between the two arms of the U-shape and is provided with three channels. The first channel leads to the right arm of the U-shaped tube and is larger in diameter than the single particle to be captured. The second channel (capture channel 16) leads to the left arm of the U-shaped tube and is smaller in diameter than the single particle to be captured. The third channel is the connecting channel 13, leading to a reservoir chamber 15 of another parallel particle capture unit 9 and is smaller in diameter than the single particle to be captured.

The capture flow channel 11 contains a plurality of particle capture unit 9 connected in series at the beginning and end. The particle capture unit 9 and the particle capture unit 8 are set symmetrically. And the particle capture unit 9 consists of a flow channel 111, a reservoir chamber 15, and a capture channel 17. Referring to Figure 3, the flow channel 111 includes a U-shaped tube, with the left arm end of the U-shaped tube of the former unit connecting to the right arm end of the U-shaped tube of the latter unit and the connection part between the U-shaped tubes being straight. The reservoir chamber 15 is located between the two arms of the U-shaped and is provided with three channels. The first channel leads to the right arm of the U-shaped tube and is larger in diameter than the single particle to be captured. The second channel (capture channel 17) leads to the left arm of the U-shaped tube and is smaller in diameter than the single particle to be captured. The third channel is the connecting channel 13, leading to the reservoir chamber 14 of another parallel particle capture unit 8, and is smaller in diameter than the single particle to be captured.

The capture flow channel 10 contains a sample inlet 1, a gas inlet 4, and an outlet 7. The capture flow channel 11 contains a sample inlet 2, a gas inlet 5, and an outlet 6.

The control layer is provided with a blocking channel 12, which is located below and perpendicular to the connection channel 13 (or in other embodiments, it may also be at a certain angle), and separated by a membrane 18.

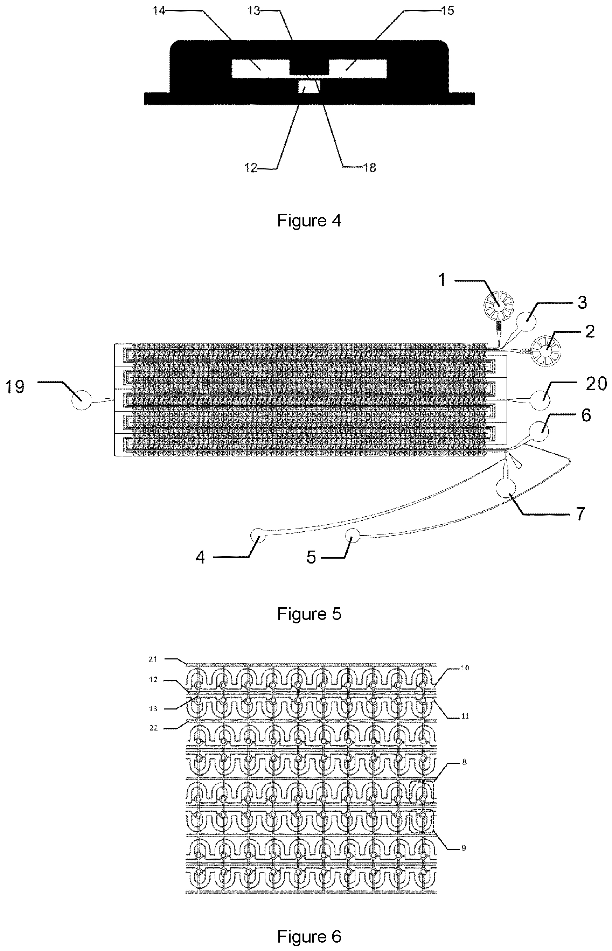

The blocking channel 12 is connected to a blocking pump, by which the pressure of the blocking channel 12 is varied to block or connect the connecting channel (see Figure 4). The blocking channel 12 is provided with an inlet 3.

As a preferred embodiment of the invention, the width of the flow channels is 60 µm, the diameter of the reservoir chambers is 100 µm; the width of the particle capture channels is 6 µm or 15 µm; the depth of the channels is 46 µm, and the number of particle capture units is 720.

As a preferred embodiment of the present invention, the particles used are A549 cells with a cell diameter of 10-20 µm.

As a preferred embodiment of the present invention, the particles used are polystyrene microspheres with a diameter of 40 µm.

As a preferred embodiment of the present invention, the polystyrene microspheres used contain mRNA capture sequence T30, which can hybridize with mRNA with polyA tails.

As a preferred embodiment of the present invention, the width of the blocking channel and the driving pump deformation chamber in the control layer is 30 µm and the height is 30 µm, respectively.

As a preferred embodiment of the present invention, all the inlets are cylindrical holes with a diameter of 1.00 mm.

As a preferred embodiment of the present invention, the material of the capture and control layers are both polydimethylsiloxane PDMS.

As a preferred embodiment of the present invention, the material of the slide is glass.

As a preferred embodiment of the present invention, both the blocking channel and the driving pump deformation chamber are filled with an aqueous solution, and the pressure thereof is controlled by controlling the driving pressure of the syringe pump.

As a preferred embodiment of the present invention, the cell flow rate used is 0.04 mL/h.

As a preferred embodiment of the present invention, the microsphere flow rate used is 0.2 mL/h.

As a preferred embodiment of the present invention, the gas phase flow rate used is 0.04 L/min.

As a preferred embodiment of the present invention, the specific workflow of the present invention is as follows:
Step A: The blocking pump is opened to increase the pressure of the blocking channel 12. The membrane 18 deforms upward. At the time the connection channel 13 is blocked. Suspension of cell A549 at the sample inlet 1 is injected with a syringe pump. When a single cell enters the particle capture unit 8, the cell first enters the reservoir chamber 14. Because the capture channel is smaller in size than that of the cell, the cell is stuck in front of the capture channel 16, while blocking the capture channel. At this point, the liquid flow rate through the reservoir chamber 14 tends to zero, and the subsequent cells cannot re-enter the particle reservoir chamber, but can only enter the next capture chamber through the flow channel 101, thus achieving a single-cell capture. High throughput single-cell capture is achieved by repeating this process in subsequent particle chambers.
Step A-1: Connection channel 13 is kept closed and the PBS solution is introduced at the sample inlet 1 to elute the excess cells from flow channel 101 out of the chip.
Step B: The tube of the sample inlet 1 is removed, and outlet 7 is plugged, with the air being introduced through the gas inlet 4 with the syringe pump system. When the air enters the particle capture chamber 8, the air enters the flow channel 101 without entering the capture channel 16 because the capture channel 16 is much smaller in size than that of the flow channel 101, with higher capillary resistance. At which time the solution in the particle reservoir chamber is retained, forming a water-in-gas micro-reaction chamber, such that each micro-reaction chamber contains a single cell, thus isolating the single cell in the reservoir chamber. When the airflow completes the chip, all single cells are isolated in single water-in-gas micro-reaction chambers separately.
Step C: A suspension of encoded microspheres (MACOSKO-2011-10 (V+), Barcoded Oligo dT primer ON Beads) is injected at a flow rate of 0.2 mL/h at the sample inlet 2 using a syringe pump. When a single microsphere enters the microsphere capture unit 9, first the microsphere enters the reservoir chamber 15. Because the capture channel diameter 17 is smaller in size than that of the microsphere, the microsphere is stuck in front of the capture channel 17, while blocking the capture channel 17. At the time, the liquid flow rate through the reservoir chamber 15 tends to zero, and subsequent microspheres cannot enter the microsphere reservoir chamber again, but only enter the next capture unit through flow channel 111, thus achieving a single microsphere capture. High throughput single microsphere capture is achieved by repeating this process in subsequent chambers.
Step C-1: The connecting channel 13 is kept closed. A cell lysate (0.2% Triton X-100) is introduced at the sample inlet 2, eluting the excess microspheres from the channel out of the chip, and replacing the solution in the capture flow channel with the cell lysate.
Step D: The tube from the particle inlet 2 is removed and outlet 6 is plugged. Air is injected at the gas inlet 5 with a syringe pump system. Because the capture channel 17 is much smaller in size than that of the flow channel 111, with a higher capillary resistance, when the air enters the particle capture unit 9, it enters the flow channel 111 without entering the capture channel 17, At this point the solution in the particle reservoir chamber is retained, forming a liquid-in-gas micro-reaction chamber, such that each micro-reaction chamber contains a single microsphere, thus isolating the individual microspheres in the reservoir chamber. When the airflow completes the chip, all the single microspheres are isolated in single water-in-gas micro-reaction chambers separately in which the droplets contain cell lysate.
Step E: The blocking channel 12 is closed, at which point the connecting channel 13 is opened. At the time the paired droplets of the encapsulated cells and microspheres are connected, the cell lysate in the microsphere reservoir chamber 15 can enter the cell reservoir chamber 14 for cell lysis by diffusion. After cell lysis, the inclusions of the cell contained in the cell lysate can also enter the microsphere reservoir chamber 15 by diffusion, where the mRNA with polyA tails in the cell inclusions can hybridize with T30 on the microsphere. After a period, sufficient exchanges of substances between the two paired chambers can be achieved for complete cell lysis and single-cell mRNA extraction. Since the paired single microspheres/single cells are isolated by FC40, there is no physical contact between the cells and microspheres, so there is no cross-contamination, and thus high throughput capture of mRNA from single cells with single microspheres can be achieved.
Step F: The capture layer is peeled off and the microspheres or cells captured in the chambers are exposed to the surface of the chip, which is eluted off with buffer solution. The extracted single-cell mRNA is subjected to subsequent analytical studies.

### Example 2

In Example 1, the substance exchanges between the two paired droplets are achieved by passive diffusion, and the exchange rate is slow. To accelerate the substance exchanges between the two paired droplets, a driving pump unit is added to the chip exemplified in Example 1 for controlling the mixing between the two droplets and controlling the distribution of the droplets between the two paired chambers.

The following is a further detailed description of the present invention in conjunction with the accompanying drawings and specific embodiments using paired capture of microspheres and cells as an example.

As shown in Figures 5-8, based on Example 1, the chip further comprises a driving pump control network channel 21, a driving pump control network channel 22, a driving pump deformation chamber 23, and a driving pump deformation chamber 24. The driving pump control network channels 21, 22 are provided with driving pump inlets 19, 20. The driving pump control network channels 21, 22 are respectively connected to the driving pump deformation chambers 23, 24, respectively. The driving pump deformation chambers 23, 24 are located at the bottom of the reservoir chambers 14, 15, respectively, and separated from the reservoir chamber 14, 15 by membranes 25 and 26.

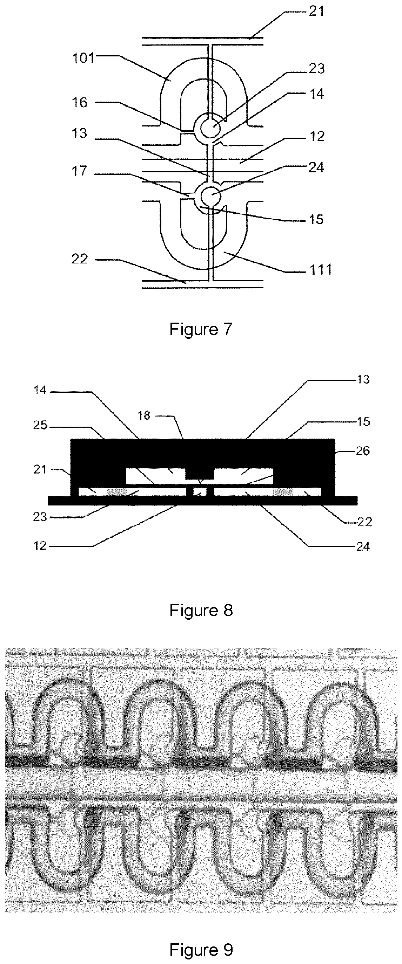

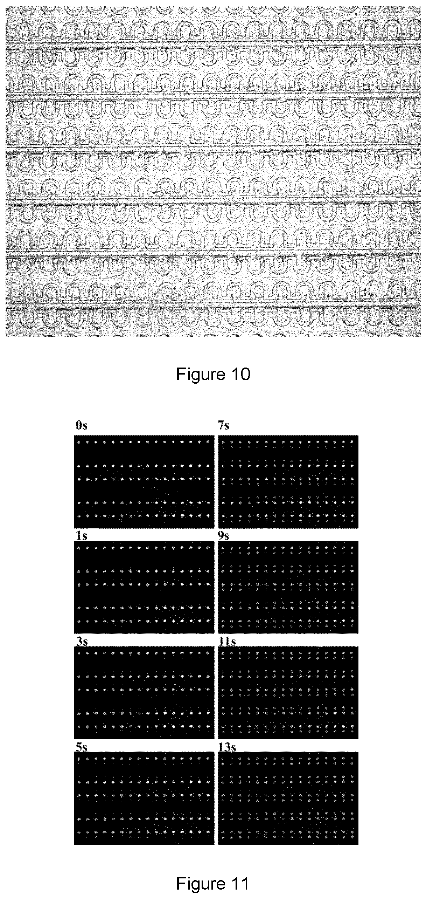

As a preferred embodiment of the present invention, the specific working process is as follows:
Based on Example 1, in step E, the blocking channel 12 is closed, at which time the connecting channel 13 is opened. At that time, the paired droplets encapsulating the cells and microspheres are connected. The pressure of the driving pump control network channel 22 is increased. When the driving pump deformation chamber 24 becomes larger, the membrane 26 protrudes upward. The volume of the particle reservoir chamber 15 is reduced and the cell lysate therein is pumped into the particle reservoir chamber 14, while the pressure of the driving pump control network channel 21 is reduced. At the time, the driving pump deformation chamber 23 is reduced, and the membrane 25 is recessed downward. The volume of the particle reservoir chamber 14 becomes larger, and into which the cell lysate in the particle reservoir chamber 15 is pumped into. When the pressure of the driving pump control network channel 22 is reduced, the driving pump deformation chamber 24 becomes smaller and the membrane 26 is recessed downward. The volume of the particle reservoir chamber 15 becomes larger, and the solution therein is pumped into the particle reservoir chamber 14; while the pressure of the driving pump control network channel 21 is increased, and the driving pump deformation chamber 23 becomes larger, the membrane 25 is recessed downward. The solution in the particle reservoir chamber 15 is pumped into the particle reservoir chamber 14. The chamber 23 becomes larger, the membrane 25 protrudes upward. The volume of the particle reservoir chamber 14 becomes smaller, and the solution therein is also pumped into the particle reservoir chamber 15. By repeating this cycle several times, a rapid exchange of substances in the particle reservoir chamber 14 and the particle reservoir chamber 15 can be achieved. The cell lysate in the particle reservoir chamber 15 can lyse the cells paired with it. After cell lysis, the released mRNA with polyA tails can enter the particle capture chamber to hybridize with T30 on the microspheres. Since the paired single microsphere/single cell is isolated by FC40, there is no physical contact between the cell and microsphere. So, there is no cross-contamination, and thus high throughput capture of mRNA from single cells with single microspheres can be achieved. Microscopic photographs of the obtained results are shown in Figures 9-11.

### Example 3

In Examples 1-2, only a single replacement of solutions or reagents in the flow channel was performed. Using the gas phase replacement method of the present invention, multiple replacements of fluids in the flow channels and independent micro-reaction chambers can also be achieved by multiple times, allowing the captured microspheres and/or cells to interact separately or simultaneously in different solutions, or to undergo chemical reactions.

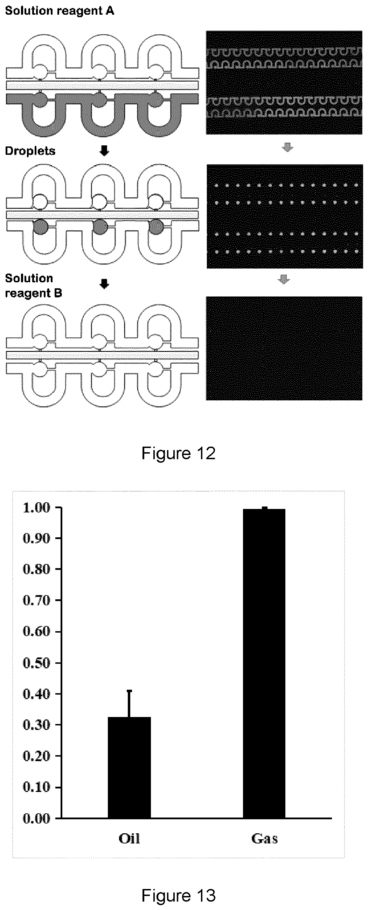

Based on Example 2, after step E, the following steps are carried out:
Step E-1: The pressure of the driving pump control network channel 22 is increased, at which point the driving pump deformation chamber 24 becomes larger, with the membrane 26 protruding upward. The volume of the particle reservoir chamber 15 is reduced, and the solution in the paired unit is all drained into the cell capture chamber. The blocking channel 12 is opened, at which time the connecting channel 13 is closed, and 1×PBS at a flow rate of 0.2 mL/h is injected at the two sample inlets 1 and 2 respectively for thoroughly washing the microspheres while preventing contamination of the microspheres by free RNA during the washing process.
Step E-2: The blocking channel 12 is closed, at which point the connecting channel 13 is opened. RT mix (1× Maxima RT buffer, 1 mM dNTPs (Clontech, Ref. 639125), 1 U/µL RNAase inhibitor (Lucigen, Ref. 30281-2), 2.5 µM Template_Switch_Oligo, and 10 U/µL Maxima H-RT (ThermoScientific, Ref. EP0751)) are injected at a flow rate of 0.2 mL/h in both sample inlets 1 and 2, to replace the solution in the capture flow channel with the RT mix. Then a step similar to step D is repeated to form water-in-air droplets in the reservoir chamber. The process is as follows: the blocking channel 12 is opened, at which point the connecting channel 13 is blocked. The tubes of sample inlet 1 and 2 are removed and the outlets 6 and 7 are plugged. Air is injected at the gas inlets 4 and 5 with a syringe pump. Liquid-in-gas micro-reaction chambers are formed in the particle capture chamber 14 and the particle capture chamber 15, respectively. Each micro-reaction chamber contains a single microsphere, which is thus isolated in the reservoir chamber. When the airflow completes the chip, all of the single microspheres are isolated separately in the single water-in-air micro-reaction chamber that contains RT mix, such that all microspheres are in RT mix solution. The microfluidic chip is placed at 42°C for 1.5h for mRNA reverse transcription, and the mRNA information within the single-cell carried on each microsphere is converted into cDNA information.
Step E-3: After the reverse transcription, TE/SDS (10 mM Tris-HCI, 1 mM EDTA, 0.1%SDS, pH=8.0), TE/TW (10 mM Tris-HCI, 1mM EDTA, 0.01% Tween, pH=8.0) and TE (10 mM Tris-HCI, 1 mM EDTA, pH=8.0) buffer are injected sequentially at the two sample inlets 1 and 2 at a flow rate of 0.2 mL/h with 20 µL of each solution to wash the microspheres. Subsequently, 20 µL of Exonuclease I mix solution (2 µL Exonuclease I (NEW ENGLAND BioLabs Inc., item no. M0293), 4 µL of 10x Exonuclease I buffer solution (NEW ENGLAND BioLabs Inc., item no. M0293), and 34 µL of ddH₂O are injected at a flow rate of 0.2 mL/h into each of the two sample inlets 1 and 2 of the chip. The chips are incubated at 37°C for 45 min, followed by the injection of 10 µL TE/SDS, 10 µL TE/TW, and 10 µL ddH₂O at the two sample inlets 1 and 2 of the chips at a flow rate of 0.2 mL/h, respectively. At the end of the whole process, nitrogen is injected into the chip at the particle gas-phase inlets 6 and 7, and combined with the micro-turbulence of the fluid inside the chip, all microspheres inside the chip are recovered nondestructively for a series of reactions such as subsequent amplification library building and sequencing. The microscopic photographs of the results are shown in Figures 12-14.

### Comparative Example 1

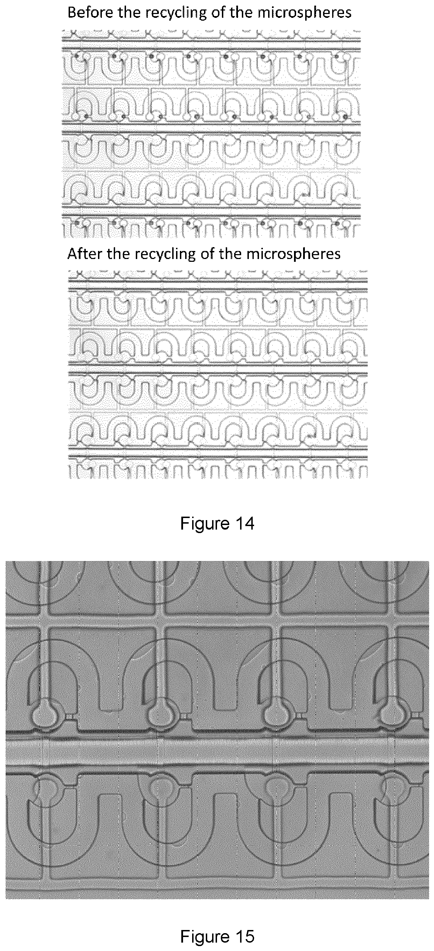

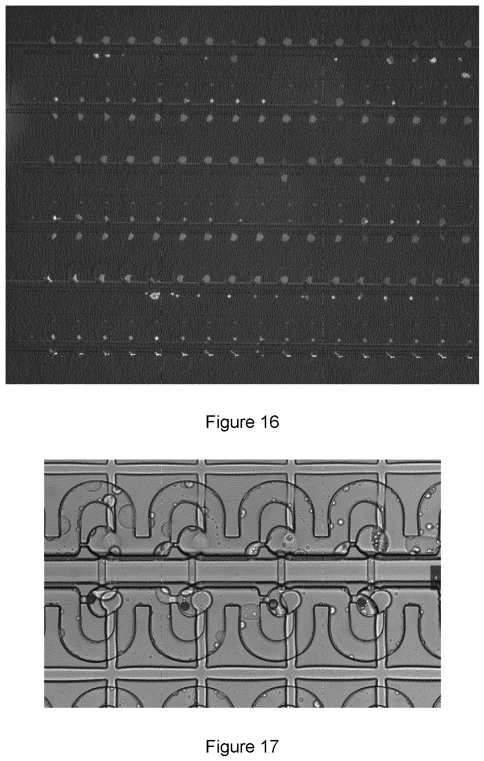

The cells and microspheres were captured and paired separately referring to the method shown in Example 2 of patent application CN107012067A, and the results obtained are shown in Figures 15-17. By using the oil phase replacement method in the prior art, stable droplets could not be formed, the paired droplets are not mixed stably, emulsion residues often existed after solvent replacement, and the recovery efficiency of microspheres was low.

## Claims

1. A method of using a microfluidic chip, comprising:
introducing a gas into the microfluidic chip to replace the liquid that has been injected into the microfluidic chip, and
forming one or more micro-reaction chambers in the form of a liquid-in-gas in the microfluidic chip.

2. The method according to claim 1, wherein the liquid that has been injected into the liquid contains or does not contain particles.

3. The method according to claim 2, wherein the particles include cells, cell clusters, microorganisms, microbial clusters, phages, exosomes, micelles, and artificial microspheres;
preferably, the artificial microspheres include polyethylene glycol, polyacrylamide, polymethacrylic acid, polymethacrylate, polyvinyl alcohol, polyethylene, polystyrene, polyester, silica, and graphene microspheres;
preferably, the artificial microspheres contain on their surface substances to achieve the intended detection purpose, including nucleic acids, nucleic acid aptamers, proteins, and polypeptides;
preferably, the artificial microspheres are microspheres modified with a nucleic acid sequence for RNA capture, modified with a nucleic acid sequence for gene capture, or modified with one or more types of molecules such as nucleic acid aptamers or antibodies.

4. The method according to any one of the preceding claims, wherein the gas comprises one or a combination of the following gases: air, nitrogen, oxygen, helium, hydrogen, carbon dioxide, neon, argon, and xenon.

5. The method according to any one of the preceding claims, wherein the gas enters the microfluidic chip through the same or different inlets as the liquid that has been injected into the microfluidic chip.

6. The method according to any of the preceding claims, wherein the gas is introduced into the microfluidic chip at a gas flow rate of 0.02 L/min to 1.00 L/min, or 0.05 L/min to 0.70 L/min;
preferably, the gas is introduced into the microfluidic chip at a gas flow rate of 0.04 L/min.

7. The method according to any of the preceding claims, wherein the time required for introducing the gas is 10 min-90 min, 10 min-40 min, or 15 min-25 min.

8. The method according to any of the preceding claims, wherein the microfluidic chip comprises a capture layer, a control layer, and a slide; wherein,
the capture layer includes two parallel sets of capture flow channels (10, 11) and a connecting channel (13) connecting them, wherein the capture flow channel (10, 11) comprises a plurality of capture unit (8, 9) in series at the beginning and end, respectively;
each capture unit comprises a flow channel (101, 111), a reservoir chamber (14, 15), and a capture channel (16, 17), respectively, and the flow channel (101, 111) comprises a U-shaped tube, with the left arm end of the U-shaped tube of the former capture unit being connected to the right arm end of the U-shaped tube of the latter capture unit;
the reservoir chamber locates between the two arms of the U-shaped tube and is provided with three channels, with the first channel leading to the fluid inlet end of the U-shaped tube and being larger in diameter than the single particle to be captured; the second channel being a captured channel, leading to the fluid outlet end of the U-shaped tube and being smaller in diameter than the single particle to be captured, the third channel being the connection channel (13), leading to the reservoir chamber of another capture unit in parallel and smaller in diameter than the single particle to be captured;
the connecting channel (13) connects the reservoir chambers (14, 15) of the two capture units (8, 9);
the capture flow channels (10, 11) are provided with a sample inlet (1, 2), a gas inlet (4, 5), and a sample outlet (6, 7), respectively;
the control layer comprises a blocking channel (12), which is located below or above the connection channel (13), intersecting with the connection channel (13), and is separated from the connection channel (13) by a membrane (18);
the blocking channel (12) is provided with an inlet (3).

9. The method according to claim 8, wherein the dimensions of the two sets of particle capture flow channels and the shape and size of the capture units therein are the same or different.

10. The method according to claim 8 or 9, wherein the flow channels have a width of 5-500 µm and a depth of 5-500 µm; the connecting channel has a width of 3-100 µm and a depth of 3-100 µm.

11. The method according to any one of claims 8-10, wherein the particles have a diameter in the range of 5-200 microns.

12. The method according to any one of claims 8-11, wherein the particles are introduced at a flow rate of 0.005 mL/h -10 mL/h.

13. The method according to any one of claims 8-12, wherein the microfluidic chip further comprises a driving pump unit for changing the volume of the reservoir chambers (14, 15), respectively, comprising connected driving pump control network channels (21, 22) and driving pump deformation chambers (23, 24); wherein the driving pump control network channels (21, 22) are further provided with driving pump inlets (19 20); the driving pump deformation chambers (23, 24) are located at the top or bottom of the reservoir chambers (14, 15), respectively, separated by a membrane (25, 26).

14. A method for obtaining an assay data, comprising:
loading a sample to be assayed into the microfluid chip and introducing a gas into the microfluid chip to form one or more micro-reaction chambers in the form of a liquid-in-gas, and obtaining the assay data by measurement;
the method for obtaining the assay data further comprises comparing the assayed data with the corresponding data stored in a database (e.g., a standard curve), and obtaining the data for quantifying the assayed sample.

15. A computer program product embodied in a computer-readable medium, when executed on a computer, the execution steps comprising:
controlling and introducing a gas into the microfluidic chip.

16. A computer program product embodied in a computer-readable medium, when executed on a computer, the execution steps comprising:
introducing a gas into the microfluidic chip loaded with a sample to be assayed, waiting for the formation of one or more micro-reaction chambers in the form of a liquid-in-gas, and then measuring and obtaining assay data for the sample.

17. A kit, comprising:
a microfluidic chip; and
a computer program product embodied in a computer-readable medium.
